# EUROPEAN PATENT APPLICATION

(11) **EP 1 719 446 A1**
(43) Date of publication of application: **08.11.2006**
(21) Application number: 05714756.3
(22) Date of filing: 24.02.2005
(51) Int. Cl.: A61B 1/04, A61B 5/00

(54) **MEDICAL WIRELESS CAPSULE-TYPE ENDOSCOPE SYSTEM**

(30) Priority: 28.02.2004 CN 200410021933
(71) Applicant: Chongqing Jinshan Science & Technology (Group) Co. Ltd., Yuzhong District Chongqing 400015 (CN)
(72) Inventor: WANG, Jinshan, Chongqing 400015 (CN); LI, Xiangdong, Chongqing 400015 (CN)
(74) Representative: Beck, Michael Rudolf
(86) International application number: PCT/CN2005/000220
(87) International publication number: WO 2005/082229

(57) **Abstract**

A medical wireless capsule-type endoscope system comprises a swallowable wireless capsule (A) and a portable image recorder (B). The capsule (A) includes image sensor (5), a microprocessor (6) for compressing image information into JPEG format, a RF transceiver module (13) and an antenna (10).The portage image recorder (B) includes an antenna (F), a RF transceiver module, and a microprocessor (14). The system also include a wireless terminal end (G) which is connected to a medical imaging workstation (E) to exchange information between the system and the medical imaging workstation (E).

## Description

### Field of Invention

The present invention relates to a medical monitoring and inspecting system, and more particularly, to a medical wireless capsule-type endoscope system which is entered into the gastrointestinal tract for endoscopically observing.

### Background of the Invention

Digestive endoscopes including gastroscopes, colonoscopes, duodenoscopes and small intestinoscopes not only have satisfactory visual field and strong controllability, but also could implement diagnostic biopsy, endoscopic ultrasonic inspection and various endoscopic treatment, such as microwave treatment, electrotomy treatment and the like. Therefore, the digestive endoscopes have become one of the most important endoscopically observing means for gastrointestinal tract diseases. However, the whole process of endoscopic check brings some pain and hurt to the subject, with the result that people fear the endoscopic check to some extent. Moreover, the existing gastroscopes, colonoscopes, duodenoscopes and small intestinoscopes could not conduct continuous complete check to the whole digestive tract except for particular parts.

An orally-taken capsule-type wireless endoscope system is disclosed in US 5,604,531. Said orally-taken capsule includes a camera system, an optical system for imaging an area of interest onto said camera system and a transmitter which transmits the video output of said camera system. The patient needs to swallow such a capsule for checking the stomach and intestines. The capsule could be orally taken unpainfully due to its small volume, and no discomfort occurs after that. The micro camera in the capsule could continually send the recorded sharp image of the inner part of the intestines and stomach to the image recording device fixed at the subject's waist through the signal transmitter. The recording device is sent to the hospital after recording, where the doctors read and analyze the image data with aid of computers. Therefore, the conditions of the intestines and stomach could be known. A capsule could work over 6 hours. Upon finishing the desired task, the capsule will be entered into large intestines with the stomach and intestines worming, and then excreted out of the body. The advantages of the capsule-type endoscope are apparent, such as small volume (presently the smallest volume of a capsule is 11.6mm×27.2mm), easy administration and simple operation. In addition, the subject need not stay in hospital, and no complication disease is generated. Furthermore, the whole digestive tract could be checked, and the image data could be repeatedly reviewed and analyzed. However, said capsule-type endoscope still has some disadvantages as follows. the M2A-type capsule endoscope of GI company of Israel with the operating modes of simplex, although it has the virtue of use handy, the effective check of capsule endoscope to the whole alimentary tract can not be achieved and the whole check process can not be controlled due to it can not adjust the sampling frequency of the capsule endoscope in real time. Therefore, some purposeful key checks can not be achieved.

### Summary of the Invention

An object of the present invention is to provide a medical wireless capsule-type endoscope system which could not only wirelessly send digital image information outside, but also wirelessly receive control commands to control the operating modes of the capsule. A pressure sensor and a temperature sensor are contained in the system, so that the operating modes could be varied by control of the pressure values detected by the pressure sensor. Furthermore, the system could transport image information to the computerized medical image workstation and receive control commands from there through wired or wireless terminals.

In order to achieve the above goals, the present invention provides a medical wireless capsule-type endoscope system including a wireless endoscope capsule and a portable image recording device. The wireless endoscope capsule includes a housing, an optical front cover connected to the housing, a light emitting diode (LED) array arranged within the housing in sequence, a lens and a power switch module. The capsule further includes an image sensor, a microprocessor, a radio frequency (RF) transceiver module and a transmit/receive antenna. The signal output of the image sensor is connected with the I/O port of the microprocessor. The image information received is transformed into the compressed JPEG format by the microprocessor and then sent to the data receiving terminal of the RF transceiver module. The information is sent to the portable image recording device via the antenna by the RF transceiver module. After the control commands received from the image recording device by the antenna are sent by the RF transceiver module to the microprocessor for processing, the control terminals for the operating modes of the LED array, the image sensor and the RF transceiver module are controlled by the I/O ports of the microprocessor. The portable image recording device includes a transmit/receive antenna array, an RF transceiver module, a microprocessor and a storage unit connected with the bus thereof. The RF transceiver module communicates the information received from the capsule by the antenna to the microprocessor by the bus or sends the information from the control terminals of the microprocessor to the capsule by the antenna.

The information from the control terminals of the microprocessor of the portable image recording device is sent to the wireless terminal of computerized medical image workstation by the RF transceiver module of the portable image recording device, or the information received from the wireless terminal of the computerized medical image workstation by the antenna array is sent by the wireless transceiver module of the portable image recording device to the microprocessor by the bus for processing, and then sent to the capsule.

The technical effects generated by the configuration of the invention are apparent. There is no fear for the subject to take the wireless endoscope capsule due to its small value and light weight. The wireless endoscope capsule takes no affect on walk and daily activity of the subject during check and is simple to operate. In addition, the subject need not stay in hospital, and no complication disease is generated. Furthermore, the whole digestive tract could be checked, and the image data could be repeatedly reviewed and analyzed by the doctors. Particularly, said wireless endoscope capsule could be controlled from outside at any moment during check. The operating modes of the wireless endoscope capsule could be managed by detection pressure. The wireless endoscope capsule system could not only wirelessly send the image of the digestive tract to the portable image recording device, but also send the temperature and pressure information of the digestive tract to the portable image recording device in real time. Moreover, the wireless endoscope capsule system could exchange information with the computerized medical image workstation by the wireless terminal.

### Brief Description of the Drawings

The present invention will become more fully understood from the detailed description given herein below for illustration only, and thus are not limitative of the present invention, and wherein:
FIG. 1 is a sectional structural view of the wireless endoscope capsule;
FIG. 2 is a schematic circuit diagram of one embodiment of the wireless endoscope capsule;
FIG. 3 is a schematic circuit diagram of the magnetic switch module of the wireless endoscope capsule 1;
FIG. 4 is a schematic view of the first embodiment of the invention;
FIG. 5 is a schematic diagram of the portable image recording device according to the first embodiment of the invention;
FIG. 6 is a schematic view of the second embodiment of the invention;
FIG. 7 is a schematic diagram of the portable image recording device according to the second embodiment of the invention;
FIG. 8 is a schematic view of the third embodiment of the invention; and
FIG. 9 is a schematic diagram of the portable image recording device according to the third embodiment of the invention.

In the above mentioned drawings:
- A:: wireless endoscope capsule
- B:: portable image recording device
- C:: storage medium
- D:: storage medium reader
- E:: computerized medical image workstation
- F:: antenna array of portable image recording device
- G:: USB wireless terminal
- H:: GPRS, CDMA, GSM, or WLAN terminal
- 1:: intestinal tract
- 2:: optical front cover
- 3:: LED array
- 4:: optical lens
- 5:: image sensor
- 6:: microprocessor
- 7:: battery
- 8:: magnetic switch module
- 9:: RF transceiver module
- 10:: antenna
- 11A:: temperature sensor
- 11B:: pressure sensor
- 12:: housing
- 13:: RF transceiver module
- 14:: microprocessor
- 15:: storage unit
- 16:: battery

### Detailed Description of the Invention

The medical wireless capsule-type endoscope system of the present invention includes a wireless endoscope capsule A, a portable image recording device B and the antenna array F thereof, a wireless terminal connected with the computerized medical image workstation E, a storage medium C and a storage medium reader D. The antenna array F includes 3-8 wireless receiving units, which is fixed around the abdomen of the subject by a specified vest. The antenna array F facilitates receiving the data sent by the wireless endoscope capsule A, and provides information for positioning the wireless endoscope capsule A in the subject body.

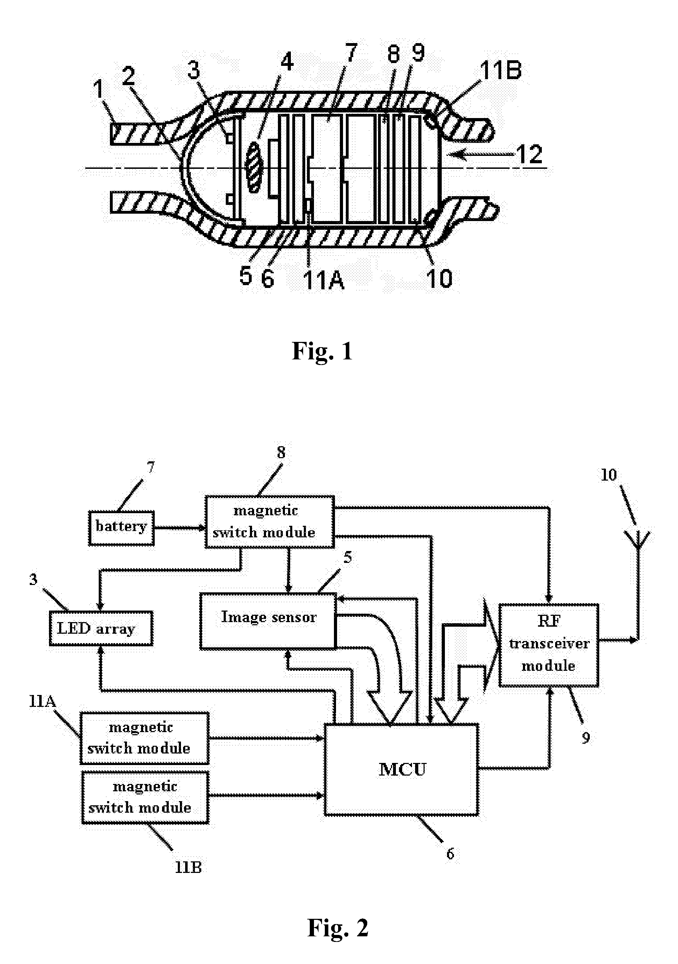

FIG.1 shows a sectional structural view of one embodiment of the wireless endoscope capsule A within the intestinal wall 1. From left to right, the wireless endoscope capsule A includes a optical front cover 2, a white light-emitting LED array 3, a lens 4, an image sensor 5, a microprocessor 6, button cells 7, a magnetic switch module 8, a RF transceiver module 9 and an antenna 10. In this embodiment, the wireless endoscope capsule A further includes a temperature sensor 11A and/or a pressure sensor 11B. A housing 12 is adhered to the optical front cover 2 as one piece. The pressure sensor 11B is closely mounted on the inner wall of the housing.

The operational principle of said system is described as follows. The white light reflected by the inner wall 1 of the stomach and intestines is passed through the optical front cover 2 and imaged on the photosensitive face of the image sensor 5 via the lens 4. Electrical signals are obtained by photoelectric conversion corresponding to the image of the inner wall 1 of the stomach and intestines. Then, the electrical signals are subjected to signal processing, such as analog to digital conversion, image signal processing and JPEG encoding and decoding, and stored in the microprocessor 6 of the wireless endoscope capsule 1. Finally, the RF transceiver module 9 sends the resulting image information out or receives command information from outside.

Referring to Fig.2, the image sensor 5 as shown could be embodied as CMOS (Complementary Metal Oxide Semiconductor) or CCD (Charged Coupled Device) image sensor, such as Image Sensor Module-VS6552 manufactured by STMicroelectronics. The microprocessor MCU 6 could use MSP340 Series chips, and the RF transceiver module could use MK70110 chips. The outputs of the temperature sensor 11A and the pressure sensor 11B are connected to the I/O ports of the microprocessor.

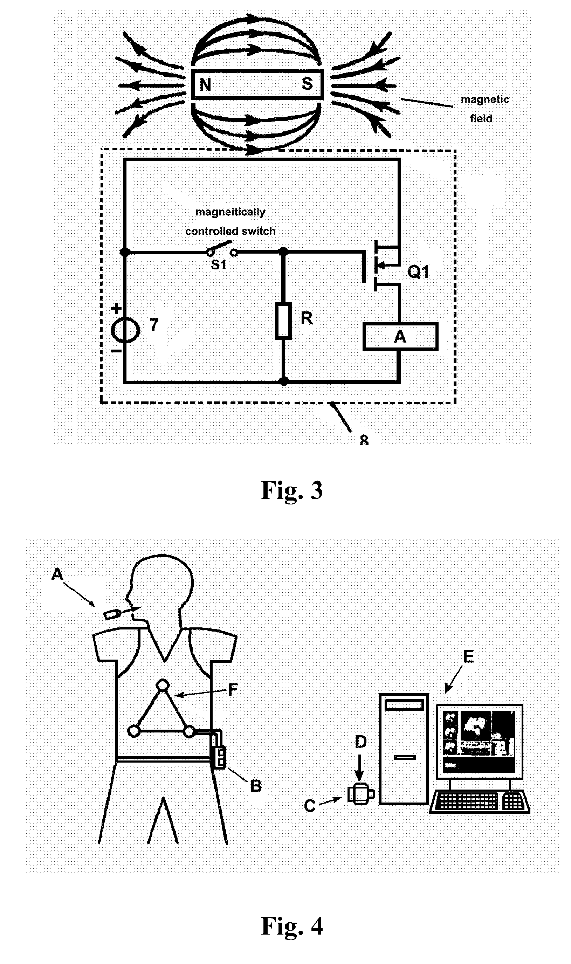

Referring to Fig.3, the magnetic switch module 8 is switched on in the magnetic field, and after the magnet is removed, it is switched off. The module 8 includes a magnetically controlled switch S1 and a field effect tube Q1. When the switch S1 is switched on, V_{GS} of the field effect tube Q1 is equal to zero which is lower than the turn-on threshold voltage of the field effect tube, so the field effect tube is turned off. In such a case, the field effect tube Q1 cuts the connection between the battery and the load circuit, and the battery can not supply power to the load circuit. On the contrary, when the switch S1 is switched on, V_{GS} is equal to the battery voltage which is higher than the turn-on threshold voltage of the field effect tube, so the field effect tube is turned on. In such a case, the battery is connected with the load circuit via the field effect tube Q1, and the battery supplies power to the load circuit.

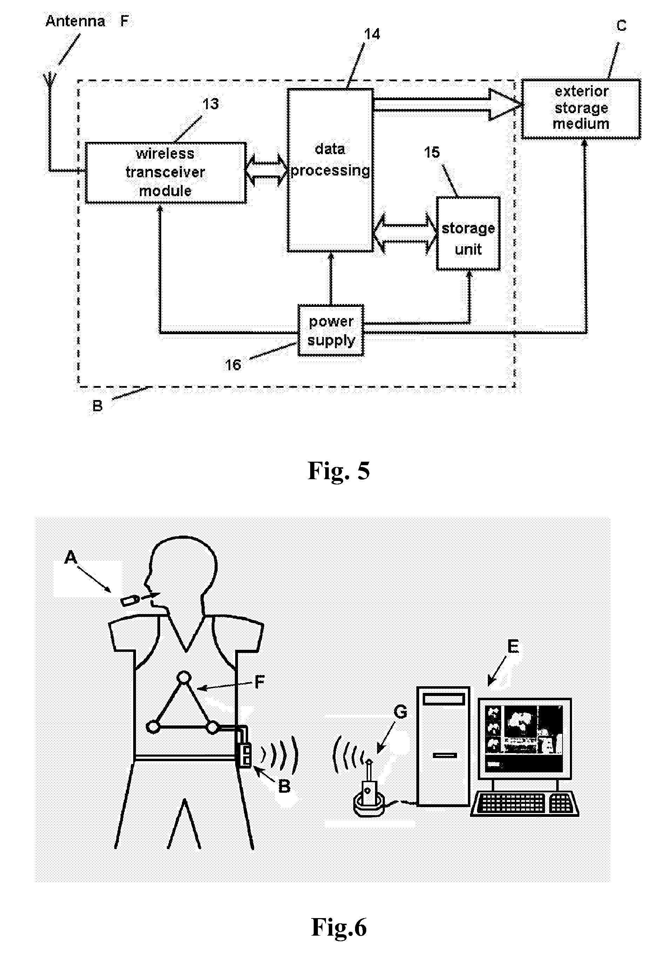

The first embodiment of the invention is shown in Figs. 4 and 5. The data exchange between the wireless endoscope capsule A powered on and the portable image recording device B should be accomplished firstly before use. The power-on process of the wireless endoscope capsule A means removing the magnet used to control the magnetic switch module and switching on the loop of the capsule by the magnetic switch module 8 in the wireless endoscope capsule A. One purpose of the data exchange between the wireless endoscope capsule A powered on and the portable image recording device B is to check whether the wireless endoscope capsule A operates in the normal condition, and the other purpose is to accomplish downloading the configuration data of the wireless endoscope capsule A to the portable image recording device B. The configuration data of the wireless endoscope capsule A refers to the specific operating modes of the wireless endoscope capsule, such as image resolution, image frame collection rate and exposure time. Whether the wireless endoscope capsule operates in the normal condition could be determined by whether the configuration data is successfully downloaded. After the configuration data is downloaded successfully, the wireless endoscope capsule enters the working state. According to the program in the microprocessor of the wireless endoscope capsule, the images are shot in real time and the temperature of the wireless endoscope capsule and the pressure applied thereon are recorded for further processing. Said processed information is sent to the portable image recording device B by way of radio frequency.

After the wireless endoscope capsule is orally taken by the subject, the micro camera system in the wireless endoscope capsule could send the image, temperature and pressure of the human digestive tract, particularly the inner wall of small intestines, to the portable image recording device B worn by the subject by way of RF transmission. The image, temperature and pressure information recorded in the storage medium C is read into the computerized medical image workstation E by the storage medium reader D for processing, displaying and analyzing.

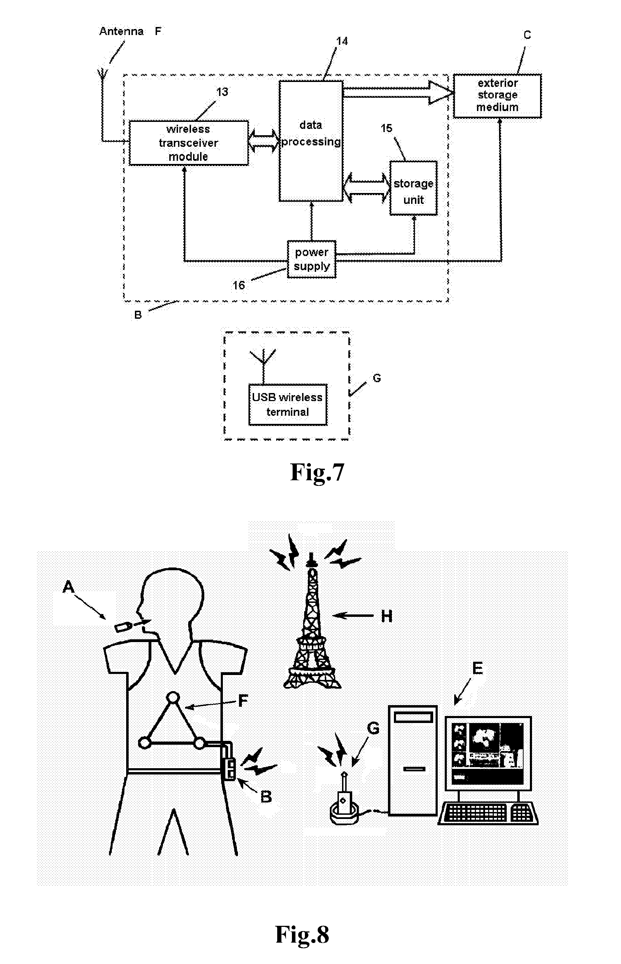

The second embodiment of the invention is shown in Figs. 6 and 7. The wireless endoscope capsule A is used in the same way as the first embodiment except the difference as follows. The information is sent and received by the portable image recording device B to and from the USB wireless terminal G connected with the computerized medical image workstation E by way of radio frequency. After the information is received by the portable recording device B, it is processed by the microprocessor and then sent to the capsule by the antenna array F.

After the wireless endoscope capsule is orally taken by the subject, the micro camera system in the wireless endoscope capsule could send the image, temperature and pressure of the digestive tract, particularly the inner wall of small intestines, to the portable image recording device B worn by the subject by way of RF transmission in a predetermined frame rate. Then, the portable image recording device B sends said data to the USB wireless terminal G connected with the computerized medical image workstation E. In such a case, the image, temperature and pressure information of the human digestive tract could be checked in real time by the doctors at the computerized medical image workstation, and optionally, the operating modes of the wireless endoscope capsule, such as image resolution, frame collection rate, exposure time, temperature sensing and pressure sensing, could be controlled in real time, so that as much as possible necessary information of the digestive tract is acquired. The RF transceiver module 13 of the invention has a multi-channel working mode with up to 125 channels, and the switching speed between different channels is lower than 200 µs. The wireless endoscope capsule system according to the second embodiment of the invention could be applied to check several patients simultaneously. Furthermore, the test data of the patients could be totally recorded in the computerized medical image workstation for further processing, displaying and analyzing.

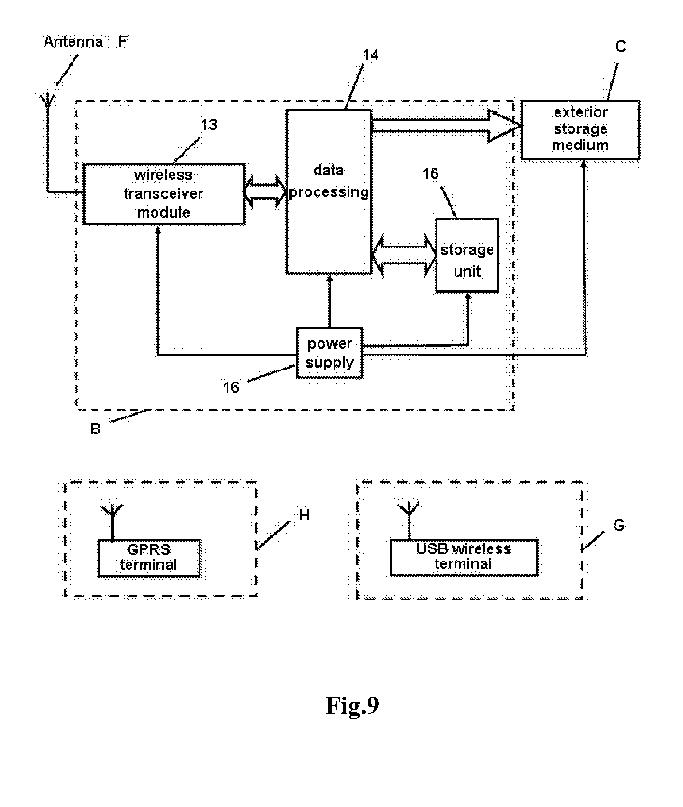

With reference to Figs. 8 and 9, the wireless endoscope capsule system according to the third embodiment of the invention combines the benefits of the first embodiment with those of the second embodiment, in which the mobile application of the wireless endoscope capsule system could be achieved by GPRS (General Packet Radio Service) mobile network. On one hand, the wireless endoscope capsule system of the third embodiment has the mobility of the first embodiment, on the other hand, it could check and control the operating modes of the wireless endoscope capsule in real time by GRPS mobile network. The data exchange between the wireless endoscope capsule powered on and the portable image recording device should be accomplished firstly before use. Then the portable image recording device exchanges data with the GPRS terminal H. The GPRS terminal H exchanges data with the USB wireless terminal G of the computerized medical image workstation E through GPRS mobile network. Finally, the information is sent to the USB wireless terminal G of the computerized medical image workstation E by way of RF transmission.

After the wireless endoscope capsule is orally taken by the subject, the micro camera system in the wireless endoscope capsule could send the image, temperature and pressure of the human digestive tract, particularly the inner wall of small intestines, to the portable image recording device worn by the subject by way of RF transmission in a predetermined frame rate. The portable image recording device exchanges the data with the GRPS terminal H. Then, the GPRS terminal sends said data to the USB wireless terminal G of the computerized medical image workstation E by GPRS mobile network. In such a case, the image, temperature and pressure information of the human digestive tract could be checked in real time by the doctors at the computerized medical image workstation, and optionally, the operating modes of the wireless endoscope capsule, such as image resolution, frame collection rate, exposure time, temperature sensing and pressure sensing, could be controlled in real time, so that as much as possible necessary information of the digestive tract is acquired. The RF transceiver module of the invention has a multi-channel working mode with up to 125 channels, and the switching speed between different channels is lower than 200 µs. The wireless endoscope capsule system according to the third embodiment of the invention could be adapted to prevent mutual interference between the wireless electrical signals emitted by the wireless endoscope capsules and the portable image recording devices worn by several subjects, thereby it could be used more freely. Therefore, several patients could be checked simultaneously, and the operating modes of the wireless endoscope capsule A within the human body could be controlled by the doctors in real time. Furthermore, the test data of the patients could be totally recorded in the computerized medical image workstation for further processing, displaying and analyzing.

## Claims

1. A medical wireless capsule-type endoscope system, comprising a wireless endoscope capsule (A) and a portable image recording device (B), the wireless endoscope capsule having a housing (12), an optical front cover (2) connected to the housing, an LED array (3) arranged within the housing in sequence, a lens (4) and a power switch module (8), **characterized in that**, the capsule further includes an image sensor (5), a microprocessor (6) for transforming the image information into a compressed JPEG format, an RF transceiver module (9) and a transmit/receive antenna (10), wherein the signal output of the image sensor is connected with the I/O port of the microprocessor, the image information received is transformed into the compressed JPEG format by the microprocessor and then sent to the data receiving terminal of the RF transceiver module, the information is sent to the portable image recording device via the antenna by the RF transceiver module after the control commands received from the image recording device by the antenna are sent by the RF transceiver module to the microprocessor for processing, the control terminals for the operating modes of the LED array, the image sensor and the RF transceiver module are controlled by the I/O ports of the microprocessor; the portable image recording device (B) includes a transmit/receive antenna array (F), an RF transceiver module (13), a microprocessor (14) and a storage unit (15) connected with the bus thereof, wherein the RF transceiver module communicates the information received from the capsule by the antenna to the microprocessor (14) by the bus or sends the information from the control terminals of the microprocessor to the capsule (A) by the antenna (F).

2. The medical wireless capsule-type endoscope system as claimed in Claim 1, **characterized in that**, a temperature sensor (11A) and/or a pressure sensor (11B) are mounted within the capsule housing, wherein the pressure sensor (11B) is closely mounted on the inner wall of the housing (12), and the outputs of the temperature sensor (11A) and the pressure sensor (11B) are connected to the I/O ports of the microprocessor (6).

3. The medical wireless capsule-type endoscope system as claimed in Claim 1, **characterized in that**, the system further includes a wireless terminal connected with the computerized medical image workstation (E); the information from the control terminals of the microprocessor of the portable image recording device is sent to the wireless terminal of the computerized medical image workstation by the RF transceiver module (13) of the portable image recording device (B); and/or the information received from the wireless terminal of the computerized medical image workstation by the antenna array is sent by the wireless transceiver module (13) of the portable image recording device (B) to the microprocessor by the bus for processing, and then sent to the capsule.

4. The medical wireless capsule-type endoscope system as claimed in Claim 3, **characterized in that**, the wireless terminal connected with the computerized medical image workstation (E) is a wireless terminal with USB ports (G) or a GPRS terminal (H), and the GPRS terminal wirelessly exchanges information with the wireless terminal with USB ports connected with the computerized medical image workstation.

5. The medical wireless capsule-type endoscope system as claimed in Claim 1 or 3, **characterized in that**, said system further includes a storage medium reader (D) wiredly connected with the computerized medical image workstation (E) and a storage medium (C), and the storage medium (C) is connected with the microprocessor (14) of the portable image recording device (B) through the socket by the bus.

6. The medical wireless capsule-type endoscope system as claimed in Claim 1, **characterized in that**, the magnetically controlled switch (S1) of the magnetic switch module (8) is switched on in the magnetic field, and after the magnet is removed, it is switched off.
